# EUROPEAN PATENT SPECIFICATION

(11) **EP 1 358 887 B1**
(45) Date of publication and mention of the grant of the patent: **17.10.2007**
(21) Application number: 02009690.5
(22) Date of filing: 29.04.2002
(51) Int. Cl.: A61K 45/06, A61K 36/258, A61K 36/23, A61K 36/481, A61K 36/484, A61K 36/539, A61K 36/718, A61K 36/899, A61P 7/02, A61P 9/10

(54) **Composition of traditional chinese medicines for preventing and treating cerebrovascular disease**
Zusammensetzung aus traditionellen chinesischen Arzneimitteln zur Prophylaxe und Behandlung von cerebrovasculären Krankheiten
Composition de médecines traditionelles Chinoises pour la prophylaxie et le traitement de maladies cérébrovasculaires

(43) Date of publication of application: 05.11.2003
(73) Proprietor: BrainGenesis Biotechnology Co., Ltd., Section 4, Taipei (TW)
(72) Inventor: Huang, Ken-Shung, BrainGenesis Biotechnology, Taipei (TW)
(74) Representative: Grünecker, Kinkeldey, Stockmair & Schwanhäusser Anwaltssozietät

(56) References cited:
- WO-A1-02/078722
- US-A- 4 842 859
- US-A- 5 589 182
- US-A- 6 123 946
- US-B1- 6 365 198
- US-B1- 6 447 814
- DATABASE WPI Week 199742 Derwent Publications Ltd., London, GB; AN 1997-453951 XP002229009 & JP 09 208479 A (KATO T), 12 August 1997 (1997-08-12)
- DATABASE WPI Week 199423 Derwent Publications Ltd., London, GB; AN 1994-185856 XP002229010 & JP 06 107555 A (KIM C), 19 April 1994 (1994-04-19)
- DATABASE WPI Week 199607 Derwent Publications Ltd., London, GB; AN 1996-065443 XP002229011 & JP 07 324039 A (TSUMURA & CO), 12 December 1995 (1995-12-12)
- DATABASE WPI Week 199719 Derwent Publications Ltd., London, GB; AN 1997-204102 XP002229012 & CN 1 097 341 A (CHE L), 18 January 1995 (1995-01-18)
- DATABASE WPI Week 199822, Derwent Publications Ltd., London, GB; AN 1998-243636, XP002229013 & IE 78 451 B2 (HALMONT MARKETING LIMITED) 11 February 1998

## Description

### Field of the Invention

The present invention relates to a composition of traditional Chinese medicines possessing activities of inhibiting platelet aggregation and prolonging bleeding time, which can be used to prevent and treat ischemic cerebrovascular disease (ischemic stroke).

### Background of the Invention

So far, certain Chinese medicines may claim to have pharmacological activity in inhibiting platelet aggregation or prolong bleeding time, yet no Chinese medicines are proved to possess significant pharmacological potential in preventing and treating ischemic cerebrovascular disease.

WO 02/78722 refers to an antineoplastic drug. Moreover, in JP19960012726 Willow compositions useful as antibacterial and antiseptic compositions are described. In document JP 07324039A reference is made to an agent for promoting production of nitrogen monoxide. JP 06107555A refers to GOOU-SEISHIN microcapsules. In document US 6, 123, 946 a hygienic bag and preparation thereof is disclosed. CN 19930112721 refers to a hat lining preparation.

### Summary of the Invention

The present invention provides a novel composition of traditional Chinese medicines.

More specifically, the present invention is related to a novel composition of traditional Chinese medicines with an activity of inhibiting platelet aggregation.

Also, the present invention is related to a novel composition of traditional Chinese medicines that can prolong bleeding time.

Further, the present invention is related to a novel composition of traditional Chinese medicines that can be used in preventing and treating cerebrovascular disease, and in particular ischemic cerebrovascular disease.

Meanwhile, the present invention also discloses a use of traditional Chinese medicines in the manufacture of a medicament for preventing and treating cerebrovascular disease in a patient, and in particular ischemic cerebrovascular disease.

The novel composition of traditional Chinese medicines of the present invention comprises at least Ren Shen, Dang Gui, Huang Qi, Gan Cao; Tian Zhu Huang and Huang Qin. These traditional Chinese medicines are translated into English according to their pronunciations in Mandarins

### Brief description of the Drawing

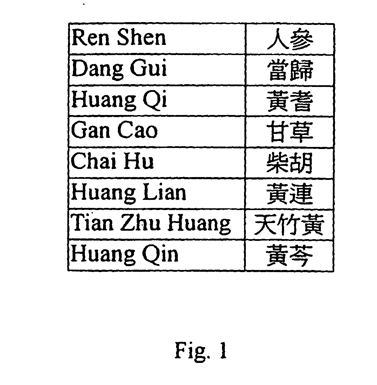

Fig. 1 shows Chinese characters of the traditional Chinese medicines used in the present invention, and their English translations according to their pronunciations in Mandarin.

### Detailed description of the Invention

The present invention provides a novel composition of traditional Chinese medicines comprising at least Ren Shen, Dang Gui, Huang Qi, Gan Cao, Tian Zhu Huang and Huang Qin. These traditional Chinese medicines are translated into English according to their pronunciations in Mandarin, and their Chinese characters are shown in Fig. 1.

Preferably, the composition of traditional Chinese medicines further comprises Chai Hu. Even more preferred the composition of traditional Chinese medicines further comprises Chai Hu and Huang Liang.

The present invention also refers to the composition of traditional Chinese medicines in the manufacture of a medicament for preventing and treating a cerebrovascular disease, preferably an ischemic cerebrovascular disease.

The composition of traditional Chinese medicines, of the present invention, was demonstrated to possess pharmaceutical activities of inhibiting platelet aggregation and prolonging bleeding time. The same composition of traditional Chinese medicines was also proven to possess a therapeutic effect on preventing and treating an ischemic cerebrovascular disease through animal experiments, and was shown to have no safety concern through safety pharmaceutical tests and subacute toxicology tests (28 days).

Preferably, the eight traditional Chinese medicines are in the form of a dry powder.

The eight traditional Chinese medicines can be obtained from the general traditional Chinese medicine store, which are processed separately, baked to dry and grinded into powder.

According to the present invention, the ratio (by weight) of the eight traditional Chinese medicines is, preferably, Ren Shen : Dang Gui : Huang Qi : Gan Cao : Chai Hu : Huang Lian : Tian Zhu Huang : Huang Qin = 140±10% : 166±10% : 180±10% : 67±10% : 140±10% : 120±10% : 120±10% : 67±10%. The amount of the one or two unused traditional Chinese medicines are zero, while the weight ratio of the others remaining unchanged, in the case when the composition of the present invention comprises six or seven traditional Chinese medicines out of the eight traditional Chinese medicines.

The composition of traditional Chinese medicines of the present invention is suitable to be administered orally.

Ren Shen (Ginseng radix) used in the present invention includes (but not limited to) Panax ginseng C. A. Meyer and Panax quinquefolium Linnaeus, and the former is preferred. Ren Shen acquired from the traditional Chinese medicine stores or trading companies is processed by steaming Ren Shen in a meshed steamer with a mixture of pure water and rice wine (3:1), which is quickly boiled with a strong fire and then with a mild flame for 90 minutes; drying and slicing the steamed Ren Shen after cooling down. Then, the Ren Shen ginseng slices are placed in an oven, and baked at 50°C for 13 hours, and grinded into powder. One thousand grams of Ren Shen (Panax ginseng C. A. Meyer) become approximately 930 grams after the processing.

Dang Gui (Angelica sinensis radix) used in the present invention includes (but not limited to) the root of Angelica sinensis (Oliv.) Diels. The unsliced Dang Gui, acquired from the traditional Chinese medicine stores or trading companies, is processed by quickly washing it with water spray until the waste water becomes clear, and then washing with distilled water. The clean Dang Gui (20 kg) is steamed in a meshed steamer by boiling a mixture of 3 kg of R.O. water, 1 kg of rice wine, and 100 gm of Cyperi Rhizoma (Cyperus rotundus L.), which is quickly boiled with a strong fire and then with a mild flame for 60 minutes, and sliced after cooling down. The Dang Gui slices are placed in an automated oven, baked for 10 hours at 55°C, and grinded into powder. One thousand grams of Dang Gui become approximately 600 grams after being processed to dry powder. According to US patent 4843067, both Levisticum officinale Koch and Angelica archangelica can be contemplated as a substitute of Dang Gui.

Huang Qi (Astragali radix) used in the present invention includes (but not limited to) the roots of Astragalus membranaceus (Fisch.) Bunge var. membranaceus, Astragalus membranaceus (Fisch.) Bunge var. mongholicus (Bunge) Hsiao, and Hedysarum polybotrys Hand.-Mazz. Huang Qi, acquired from traditional Chinese medicine stores or trading companies, is processed, which comprises washing Huang Qi with water, slicing, drying it by baking it in an oven at 50°C for 16 hours, and grinding the dried slices into powder. One thousand grams of Huang Qi become approximately 650 grams after the processing.

Gan Cao used in the present invention is the rhizome of Glycyrrhiza uralensis Fisch., Glycyrrhiza glabra L. or Glycyrrhiza inflata Bat, and preferably Glycyrrhiza uralensis Fisch. Gan Cao, acquired from traditional Chinese medicine stores or suppliers, is processed by water-washing, slicing, baking at 50°C for 12 hours, and, grinding into powder. One thousand grams of Gan Cao become approximately 720 grams after the processing.

Chai Hu (Bupleurum radix) used in the present invention is the roots of Bupleurm chinensis DC, Bupleurum scorzonerifolium Willd., and other plants in the same genus, and preferably is the root of Bupleurm chinensis DC. Chai Hu, acquired from traditional Chinese medicine stores or suppliers, is processed by water-washing, drying by baking for 12 hours, and grinding into powder. One thousand grams of Chai Hu become approximately 720 grams after the processing.

Huang Lian used in the present invention is rhizome of Coptis chinensis Franch., Coptis deltoidea C. Y. Cheng et Hsiao, Coptis teetoides C. Y. Cheng, and other plants in the same genus. Huang Lian, acquired from traditional Chinese medicine stores or suppliers, is processed by water-washing, baking at 50°C for 12 hours, and grinding into-powder. One thousand grams of Huang Lian become approximately 750 grams after the processing.

Tian Zhu Huang (Bambusae concretio silicea) used in the present invention is a bulk accumulated between nodes of a stem of Phylllostachys nigra MUNRO var. henonis STAPF ex RENDLE) and other plants in the same genus. Tian Zhu Huang, acquired from traditional Chinese medicine stores or suppliers, is screened by choosing those floating on water, which is baked at 55°C for 18 hours, and grinded into powder. One thousand grams of Tian Zhu Huang become approximately 850 grams after the processing.

Huang Qin (Scutellariae radix) used in the present invention is the root of Scutellaria baicalensis Georgi. Huang Qin, acquired from traditional Chinese medicine stores or suppliers, is processed by washing it with water. The clean Huang Qin (10 kg) is steamed in a meshed steamer by boiling a mixture of 3 kg of R.O. water and 100 gm of Corni Fructus, which is quickly boiled with a strong fire and then with a mild flame for 60 minutes, and sliced after cooling down. The Huang Qin slices are placed in an automated oven, baked for 11 hours at 50°C, and grinded into powder. One thousand grams of Huang Qin become approximately 700 grams after the processing.

### Preparation Example:

Panax ginseng, Dang Gui, Huang Qi, Gan Cao, Chai Hu, Huang Lian, Tian Zhu Huang, Huang Qin were purchased from traditional Chinese medicine suppliers. Based on the source identification, Panax ginseng belonged to Panax ginseng C. A. Meyer, Dang Gui belonged to Angelica sinensis (Oliv.) Diels, Huang Qi belonged to Hedysarum polybotrys Hand.-Mazz., Gan Cao belonged to Glycyrrhiza uralensis Fisch., Chai Hu belonged to Bupleurum longesadiatum Turca., Huang Lian belonged to Coptis chinensis Franch., Tian Zhu Huang was from Bambusa textilis McCluture; Schizostachyum chinense Rendle, and Huang Qin belonged to Scutellaria baicalensis Georgi. These eight traditional Chinese medicines were processed according to the processing steps described above, the resulting dry powders were sieved with a mesh No. 10 sieve, and the penetrated portions were used.

The eight traditional Chinese medicines in the powder form were mixed according to the following weight ratio: Panax ginseng : Dang Gui : Huang Qi : Gan Cao : Chai Hu : Huang Lian : Tian Zhu Huang : Huang Qin = 140 : 166 : 180 : 67 : 140 : 120 : 120 : 67, and a powder pharmaceutical composition was obtained, which is named as BNG-1.

### Example 1:

### 1. Test substance and Dosing pattern

BNG-1 was provided by BrainGenesis Biotechnology Co., Ltd., Taiwan, and dissolved in distilled water. For oral administration (PO) in *in vivo* testing, the dosage is 20 ml/kg for mice. Tested animals received an initial dosage of 1000 mg/kg daily for 8 consecutive days. For *in vitro* assays, 0.1 ml of testing material was added to the 10 ml bath to give a final concentration of 1000 µg/ml, and incubated with isolated tissues for 5 minutes before evaluation of possible agonist response or challenge with agonists in the Arachidonic Acid Platelet Aggregation.

### 2. Animals

In these studies, male/female ICR derived mice, and New Zealand derived albino male/female rabbits provided by animal breeding center of MDS Panlabs Taiwan, Ltd. were used. All animals were maintained in a controlled temperature (22°C-24°C) and humidity (60%-80%) environment with 12 hours light dark cycles for at least one week in MDS Panlabs Taiwan laboratory prior to be used.

### 3. Method:

### Arachidonic Acid, Platelet Aggregation Agonism/Antagonism

Venous blood obtained from male or female New Zealand derived albino rabbits weighing 2.5-3 kg was used. Blood sample was mixed with one-tenth volume of trisodium citrate (0.13 M) and centrifuged at room temperature for 10 min at 220 g. Testing substance by volume of 0.025 ml was added to the 0.45 ml tissue bath to get a testing concentration of 1000 µg/mL. The aggregation of the platelet enriched plasma (6 x 10⁸ platelets/ml) by 50 percent or more ( ≧ 50%) within 5 minutes, relative to 100 µM Arachidonic acid control response at 37°C as measured by an optical aggregometer, indicated possible Arachidonic acid receptor agonist activity. At a test substance concentration where no significant agonist activity was seen, the ability to reduce the Arachidonic acid-induced maximum non-reversible aggregation response by 50 percent or more (≥ 50%) indicated Arachidonic acid receptor antagonist activity. Each concentration was tested two separate preparations.

The concentration of 50% inhibition of aggregation induced by 100 µM Arachidonic acid (IC₅₀) of BNG-1 was also determined, which is 176.5 µg/ml.

### Bleeding Time (PO)

A group of 5 ICR derived male mice weighing 22 ± 2 g was administered PO at the dosage of 1000 mg/kg daily for seven days and one dosage of 1000 mg/kg was administered orally one hour before standardized transection of the tip (0.5 mm) of each tail on the eighth day. The mice, in holders, were immediately suspended vertically with the distal 2 cm of each tail immersed in a test tube containing saline at 37° C. The time required for bleeding to cease over a period of 15 seconds was then determined. A maximun cut-off time of 180 seconds was set. Prolongation of bleeding time by 50 percent or more (≧50%) relative to a control group of animals was considered significant.

### 4. Results:

| | | | | |
|---|---|---|---|---|
| AA-platelet Agg - antag | *in vitro* | 1000 µg/ml | 100% | n=2 |
| AA-platelet Agg - antag | *in vitro* | 300 µg/ml | 100% | n=2 |
| AA-platelet Agg - antag | *in vitro* | 100 µg/ml | 0% | n=2 |
| Bleeding time | PO | 1000 mg/kg x 8 | 60% | n=5 |
| Bleeding time | PO | 1000 mg/kg x 8 | 63% | repeat n=5 |
| Bleeding time | PO | 300 mg/kg x 8 | 0% | n=5 |

### Control Example 1 :

BNG-4, BNG-5, BNG-6 and BNG-7 were prepared similarly as BNG-1 in Preparation Example, except that some of the eight Chinese herb medicines of BNG-1 were omitted while the weight proportions remaining the same.

The procedures of Arachidonic Acid, Platelet Aggregation Agonism/Antagonism test method in Example 1 were repeated to determine IC₅₀ of BNG-4, BNG-5, BNG-6 and BNG-7. The formulas and results of BNG-4, BNG-5, BNG-6 and BNG-7 are shown as follows in comparison with IC₅₀ of BNG-1.

| Test substance | IC₅₀ (concentration of 50% inhibition of aggregation induced by 100 µM Arachidonic acid) |
|---|---|
| BNG-1 | 176.5 µg/ml |
| BNG-4 (Dang Gui and Huang Qi omitted) | 406.6 µg/ml |
| BNG-5 (Dang Gui and Chai Hu omitted) | 379.2 µg/ml |
| BNG-6 (Huang Qin and Huang Lian omitted) | 393.7 µg/ml |
| BNG-7 (Dang Gui, Chai Hu, Huang Qin and Huang Lian omitted) | >1000 µg/ml |

### Example 2:

### 1. Test Substances and Dosing Pattern

### Group 1 (5 rats): Vehicle Control

Saline was given orally (PO) in a dosage of 10 mL/kg immediately after MCAO and day 1, then every 24 hours for 7 consecutive days totally. Group 2 (5 rats): Positive Control (MK-801)

MK-801 was given by intraperitoneally (IP) injection at a dose of 0.3 mg/kg in a dosage of 5 mL/kg at 0, 6, 24, 30, 48 and 54 hours after MCAO. Group 3 (5 rats): BNG-1 treatment

BNG-1 dissolved in saline was given orally (PO) at a dosage of 1000 mg/kg in a dosing volume of 10 mL/kg immediately after MCAO on day 1, then every 24 hours for 7 consecutive days in total.

### 2. Animals

Male Sprauge Dawely rats weighting 180-240 g (10 weeks of age) from the Animal Resources Center, Medical College of National Taiwan University were used. The animals were maintained in a controlled temperature (22°C-24°C) and humidity (60%-80%) environment with 12 hour light dark cycles (6:00 a.m./6:00 p.m.) for at least one week in MDS Panlabs Taiwan, Ltd laboratory prior to use.

### 3. Equipment

Dental drill (UPOWER UG 33, SELECTOR-M), Image Analyzer (Life Science Resources VISTA Version 3.0), Infant Incubator (Brighten Life BL-90-SC), Magnifying stereomicroscope (ZEISS, Stemi 1000), Microscissors (A. Heiss), Microtome (SHANDON, Varistain 24-4 Automatic Slide, U.K.) and Rectal thermistor probe (Harvard Homeothermic Blanket Control Unit) were used.

### 4. METHODS

### Brain Ischemia, Middle Cerebral Artery Occlusion (MCAO)

Permanent brain ischemia via middle cerebral artery occlusion (MCAO) was carried out under chloral hydrate (500 mg/10 mL/kg IP) anesthetized. The temporoparietal region was shaved and a skin incision was made between the lateral aspect of the orbit and the external acoustic meatus. The superior pole of the parotid gland was reflected downwards as was the temporalis muscle after partial resection of its cranial insertion. The distal course of the middle cerebral artery was then visible through the translucent skull.

Under a 10 x magnifying stereomicroscope, craniectomy was performed with a dental drill and then enlarged with fine synovectomy rongeurs. The middle cerebral artery at the proximal site of branch originated from interior carotid artery was cut with a microscissors after which the temporalis muscle and parotid gland were replaced. The incision was lightly dusted with kanamycin, the scalp was sutured and a 10% povidone iodine solution was topically applied. During surgery, the animals were maintained normothermic by means of a homeothermic heating system coupled to a rectal thermistor probe. Under these conditions, rectal temperature was maintained within physiological limits (37.5 ± 1.0°C). After surgery, animals were kept in infant incubator (37.5 ± 1.0°C) while recovering from anesthesia for 1 hour. After recovery, the rats were housed 5 per cage with free access to food and water and kept in a clean animal room (23.0 ± 10°C).

BNG-1, dissolved in saline as vehicle, was administered at doses of 1000 mg/kg (n=5) orally (PO) in a dosing volume of 10 ml/kg daily for 7 consecutively days started immediately after MCAO. The vehicle-control group (n=5) was similarly treated with saline alone. The positive control reference agent MK-801 (RBI, Natick, MA 01760-2447, US) dissolved with saline was injected at a dose of 0.3 mg/kg intraperitoneally (IP) in a dosing volume of 5 ml/kg at 0, 6, 24, 30, 48 and 54 hours after MCAO.

On the eighth day after the ischemic insult, all animals were sacrificed by decapitation. Their brains were rapidly removed and immediately frozen at -70°C in deep freezer (NUAIR^{™}, NU-6511). Twenty-four hours later, whole brain coronal sections (30 µm) were obtained by use of a microtome ("SHANDON" Varistain 24-4 Automatic Slide). Every 13^{th} section (i.e. 390 µm apart) totally covering the infarction area of 12 mm length was selected for histological examination. Altogether 30 slices, stained by 2% cresyl violet, were used to measure the area of ischemic damage. This was quantitatively assessed by an Image Analyzer. The total ischemic area (mm²) of each coronal slice from each animal was summated and expressed as the mean ± SEM. The calculated infarcted volume (mm³) of mm² x specific distance (390 µm) was then expressed as the mean ± SEM for each experiment group. The effect of BNG-1 and MK-801 treatment was calculated for comparison with the vehicle control group by means of the unpaired Student's t test, differences were considered significant at * P < 0.05. For each animal, body temperature was recorded before 0 minutes (pre-dosage) and 30 minutes (post-dosage) after each oral administration. Tukey multiple comparison test was applied for comparison between pre-dosage and post-dosage temperatures.

### 5. Results

BNG-1, evaluated at a dose of 1000 mg/kg PO for 7 consecutive days post-treatment, significantly reduced (46.29%) brain ischemia in rats subjected to unilateral and permanent middle cerebral artery occlusion. No toxicity or mortality was observed in tested animals during the 7 consecutive days study. No significant change in body temperature was observed. MK-801 significantly reduced (48.38%) brain ischemia.

### Example 3:

Permanent brain ischemia via middle cerebral artery occlusion (MCAO) was carried out to Male Sprange Dawely rats by the same procedures as in Example 2.

BNG-1, dissolved saline as vehicle, was administered at doses of 1000 mg/kg and 500 mg/kg orally (PO) for 7 days daily before and for 3 days daily after MCAO. The vehicle-control group was similarly treated with saline alone. The positive control reference agent MK-801 was injected intraperitoneally (IP) at a dose of 0.3 mg/5 ml/kg immediately after MCAO 0, and again after 6, 24, 30, 48 and 54 hrs. On the fourth day the ischemic insult, all animals were sacrificed by decapitaiton. The total ischemic area of their brains were determined according to the same procedures as in Example 2.

Under the experimental conditions used, MCAO caused a reproducible ischemia of around 50%-60% of the affected hemisphere. For the most part, the area of damage was largely confined to various cortical regions (i.e. frontal, sensorimotor, auditory and occipital cortices) and only rarely involved damage to components of the basal ganglia. Relative to the vehicle-treated control group, MK-801 significantly reduced the total infarcted volume by 66.30 ± 10.50% at a dose of 0.3 mg/kg IP x 6. At the 1000 mg/kg PO x 10 dose level, BNG-1 significantly reduced the total infarcted volume of 44.09 ± 9.01% while a non-significant 14.17 ± 19.43% reduction was observed after the 500 mg/kg x 10. None of the compounds caused any significant change in body temperature.

The safety pharmacology® of BNG-1 was evaluated according to the safety pharmacology® testing package (non-GLP) undertaken at MDS Panlabs Taiwan, Ltd., Taiwan.

**Safety Pharmacology® test results**

| Experiments | Administration route | Dosage or concentration | Activity |
|---|---|---|---|
| Mice behavior reaction-Irwin observation screening (General behavioral, autonomic, neurological sign, etc, 38 tests and 7 days toxicity lethal rate observation) | oral | 0.5, 1.0 and 2.0 (gm/kg) | No effect |
| Central nervous system (spontaneous activity motor in coordination, prolongation of hexobarbital sleeping time, protection against maximal-electroshock or pentylenetetrazole-induced convulsions and mortality, proconvulsant or anticonvulsant response and analgesic activity (tail flick response and phenylquinone-induced writhing) in mice and body temperature in rats) | oral | 0.5, 1.0 and 2.0 (gm/kg) | No effect |
| Respiration-circulation system (Alteration of mean, systolic and diastolic blood pressure, femoral blood flow, heart rate, QT interval, PR interval, QRS duration and S-T segment and respiratory rate in dogs) | oral | 1 (gm/kg) | No effect |
| Urine volume output, electrolyte excretion and urinary pH values in rats | oral | 1 (gm/kg) | No effect |
| Gastrointestinal system (Gastrointestinal Motility in mice) | oral | 1 (gm/kg) | No effect |
| Contractile responses of guinea pig ileum induced by Acetylcholine, Histamine, and Barium Chloride | In vitro | 1 (mg/ml) | Reduced contractile |
| Contractile responses of guinea pig ileum induced by Acetylcholine | In vitro | 0.3 (mg/ml) | Reduced contractile |

The result suggests that it should not have safety concern when orally applying 1 gm/kg of BNG-1 to rats.

### The acute toxicity test of BNG-1

Singe dosage of BNG-1 viscous suspension (5gm/kg) was orally apply to 6 male and 6 female rats, control groups were applied with control solution without test material(1%CMC solution), and the acute toxicity of BNG-1 to rats was determined. Each rat was administered with twice (2 hours interval) of BNG-1 or control solution, undergo clinical observation for 14 days. None of them show any clinical toxicity symptom, and no symptom was observed either in organs or tissues by bare eyes after dissecting the rats. Hence, with 5gm/kg dosage of BNG-1, it did not cause any observable acute toxicity in rats. Therefore, 5 gm/kg is the "no observable effect level" (NOEL) for BNG-1, and can be classified as practically nontoxic material.

To summarize the test results obtained in the above, it suggests that BNG-1, in 1 gm/kg, can prevent and treat ischemic cerebrovascular disease in mice; and in that dosage, it should not has any safety concern, and it does not show acute toxicity. These results evidence that BNG-1 has a great potential of preventing and treating ischemic cerebrovascular disease in human.

It also has been found that, under *in vitro* administration with 1 mg/ml of BNG-1, 67% relaxation activity against the spontaneous tension of respiratory tract in guinea pig ileum was observed, and 31% enhancement in contraction force of myocardium of guinea pig ileum was also observed.

## Claims

1. A composition of traditional Chinese medicines comprising at least Ren Shen, Dang Gui, Huang Qi, Gan Cao, Tian Zhu Huang and Huang Qin.

2. The composition according to claim 1 further comprising Chai Hu.

3. The composition according to claim 2 further comprising Huang Lian.

4. The composition according to claims 1, 2 or 3, wherein said traditional Chinese medicines are in the form of a dry powder.

5. The composition according to claim 4, wherein said traditional Chinese medicines are in a weight ratio of Ren Shen : Dang Gui : Huang Qi : Gan Cao : Chai Hu : Huang Lian : Tian Zhu Huang : Huang Qin = 140±10% : 166±10% : 180±10% : 67±10% : 140±10% : 120±10%: 120±10% : 67±10%, wherein the amounts of one or two of said traditional Chinese medicines are zero, while the weight ratio of the others remain unchanged, in the case when said composition comprise six or seven of said eight traditional Chinese medicines.

6. A pharmaceutical composition for inhibiting platelet aggregation comprising at least the traditional Chinese medicines Ren Shen, Dang Gui, Huang Qi, Gan Cao, Tian Zhu Huang and Huang Qin as active ingredients.

7. The pharmaceutical composition according to claim 6 further comprising Chai Hu.

8. The pharmaceutical composition according to claim 7 further comprising Huang Lian.

9. The pharmaceutical composition according to claims 6, 7 or 8, wherein said traditional Chinese medicines are in the form of a dry powder.

10. The pharmaceutical composition according to claim 9, wherein said traditional Chinese medicines are in a weight ratio of Ren Shen : Dang Gui : Huang Qi : Gan Cao : Chai Hu : Huang Lian : Tian Zhu Huang : Huang Qin = 140±10% : 166±10%: 180±10% : 67±10%: 140±10%: 120±10% : 120±10%: 67±10%, wherein the amounts of one or two of said traditional Chinese medicines are zero, while the weight ratio of the others remain unchanged, in the case when said composition comprise six or seven of said eight traditional Chinese medicines.

11. A pharmaceutical composition for prolonging bleeding time in a patient comprising at least the traditional Chinese medicines Ren Shen, Dang Gui, Huang Qi, Gan Cao, Tian Zhu Huang and Huang Qin as active ingredients.

12. The pharmaceutical composition according to claim 11 further comprising Chai Hu.

13. The pharmaceutical composition according to claim 12 further comprising Huang Lian.

14. The pharmaceutical composition according to claims 11, 12 or 13, wherein said traditional Chinese medicines are in the form of a dry powder.

15. The pharmaceutical composition according to claim 14, wherein said traditional Chinese medicines are in a weight ratio of Ren Shen : Dang Gui : Huang Qi : Gan Cao : Chai Hu : Huang Lian : Tian Zhu Huang : Huang Qin = 140±10% : 166±10% : 180±10% : 67±10% : 140±10% : 120±10% : 120±10% : 67±10%, wherein the amounts of one or two of said traditional Chinese medicines are zero, while the weight ratio of the others remain unchanged, in the case when said composition comprise six or seven of said eight traditional Chinese medicines.

16. A pharmaceutical composition for preventing and treating a cerebrovascular disease in a patient comprising at least the traditional Chinese medicines Ren Shen, Dang Gui, Huang Qi, Gan Cao, Tian Zhu Huang and Huang Qin as active ingredients.

17. The pharmaceutical composition according to claim 16 further comprising Chai Hu.

18. The pharmaceutical composition according to claim 17 further comprising Huang Lian.

19. The pharmaceutical composition according to claim 16, 17 or 18, wherein said traditional Chinese medicines are in the form of a dry powder.

20. The pharmaceutical composition according to claim 19, wherein said traditional Chinese medicines are in a weight ratio of Ren Shen : Dang Gui : Huang Qi : Gan Cao : Chai Hu : Huang Lian : Tian Zhu Huang : Huang Qin = 140±10% : 166±10% : 180±10% : 67±10% : 140±10% : 120±10% : 120±10% : 67±10%, wherein the amounts of one or two of said traditional Chinese medicines are zero, while the weight ratio of the others remain unchanged, in the case when said composition comprise six or seven of said eight traditional Chinese medicines.

21. The pharmaceutical composition according to claim 16, wherein said cerebrovascular disease in an ischemic cerebrovascular disease.

22. The composition according to claims 5, 10, 15 or 20 which is to be administered orally.

23. Use of traditional Chinese medicines in the manufacture of a medicament for preventing and treating cerebrovascular disease, said traditional Chinese medicines comprising at least six of the following eight traditional Chinese medicines: Ren Shen, Dang Gui, Huang Qi, Gan Cao, Chai Hu, Huang Lian, Tian Zhu Huang, Huang Qin.

24. The use according to claim 23, wherein said traditional Chinese medicines comprise at least seven of said eight traditional Chinese medicines.

25. The use according to claim 24, wherein said traditional Chinese medicines comprise said eight traditional Chinese medicines.

26. The use according to claim 23, 24 or 25, wherein said eight traditional Chinese medicines are in the form of a dry powder.

27. The use according to claim 26, wherein said traditional Chinese medicines are in a weight ratio of Ren Shen : Dang Gui : Huang Qi : Gan Cao : Chai Hu : Huang Lian : Tian Zhu Huang : Huang Qin = 140±10% : 166±10%: 180±10% : 67±10% : 140±10% : 120±10% : 120±10% : 67±10%, wherein the amounts of one or two of said traditional Chinese medicines are zero, while the weight ratio of the others remain unchanged, in the case when said composition comprise six or seven of said eight traditional Chinese medicines.

28. The use according to claim 23, wherein said cerebrovascular disease is an ischemic cerebrovascular disease.

29. The use according to claim 28, wherein said medicament is to be administered orally.

## Patentansprüche

1. Zusammensetzung von traditionellen chinesischen Arzneimitteln, umfassend mindestens Ren Shen, Dang Gui, Huang Qi, Gan Cao, Tian Zhu Huang und Huang Qin.

2. Zusammensetzung nach Anspruch 1, außerdem umfassend Chai Hu.

3. Zusammensetzung nach Anspruch 2, außerdem umfassend Huang Lian.

4. Zusammensetzung nach den Ansprüchen 1, 2 oder 3, wobei die traditionellen chinesischen Arzneimittel in Form eines trockenen Pulvers vorliegen.

5. Zusammensetzung nach Anspruch 4, wobei die traditionellen chinesischen Arzneimittel in einem Gewichtsverhältnis von Ren Shen : Dang Gui : Huang Qi: Gan Cao : Chai Hu : Huang Lian : Tian Zhu Huang : Huang Qin = 140±10 % : 166±10 % : 180±10 % : 67±10 %: 140±10 % : 120±10 % : 120±10 % : 67±10 % vorliegen, wobei die Mengen von einem oder zwei der traditionellen chinesischen Arzneimittel Null sind, während das Gewichtsverhältnis der anderen unverändert bleibt, falls die Zusammensetzung sechs oder sieben der acht traditionellen chinesischen Arzneimittel umfasst.

6. Pharmazeutische Zusammensetzung zum Hemmen der Plättchenaggregation, umfassend mindestens die traditionellen chinesischen Arzneimittel Ren Shen, Dang Gui, Huang Qi, Gan Cao, Tian Zhu Huang und Huang Qin als Wirkstoffe.

7. Pharmazeutische Zusammensetzung nach Anspruch 6, außerdem umfassend Chai Hu.

8. Pharmazeutische Zusammensetzung nach Anspruch 7, außerdem umfassend Huang Lian.

9. Pharmazeutische Zusammensetzung nach den Ansprüchen 6, 7 oder 8, wobei die traditionellen chinesischen Arzneimittel in Form eines trockenen Pulvers vorliegen.

10. Pharmazeutische Zusammensetzung nach Anspruch 9, wobei die traditionellen chinesischen Arzneimittel in einem Gewichtsverhältnis von Ren Shen : Dang Gui : Huang Qi : Gan Cao : Chai Hu : Huang Lian : Tian Zhu Huang : Huang Qin = 140±10 % : 166±10 % : 180±10 % : 67±10 %: 140±10 % : 120±10 % : 120±10 % : 67±10 % vorliegen, wobei die Mengen von einem oder zwei der traditionellen chinesischen Arzneimittel Null sind, während das Gewichtsverhältnis der anderen unverändert bleibt, falls die Zusammensetzung sechs oder sieben der acht traditionellen chinesischen Arzneimittel umfasst.

11. Pharmazeutische Zusammensetzung zum Verlängern der Blutungszeit in einem Patienten, umfassend mindestens die traditionellen chinesischen Arzneimittel Ren Shen, Dang Gui, Huang Qi, Gan Cao, Tian Zhu Huang und Huang Qin als Wirkstoffe.

12. Pharmazeutische Zusammensetzung nach Anspruch 11, außerdem umfassend Chai Hu.

13. Pharmazeutische Zusammensetzung nach Anspruch 12, außerdem umfassend Huang Lian.

14. Pharmazeutische Zusammensetzung nach den Ansprüchen 11, 12 oder 13, wobei die traditionellen chinesischen Arzneimittel in Form eines trockenen Pulvers vorliegen.

15. Pharmazeutische Zusammensetzung nach Anspruch 14, wobei die traditionellen chinesischen Arzneimittel in einem Gewichtsverhältnis von Ren Shen : Dang Gui : Huang Qi : Gan Cao : Chai Hu : Huang Lian : Tian Zhu Huang : Huang Qin = 140±10 % : 166±10 % : 180±10 % : 67±10 %: 140±10 % : 120±10 % : 120±10 % : 67±10 % vorliegen, wobei die Mengen von einem oder zwei der traditionellen chinesischen Arzneimittel Null sind, während das Gewichtsverhältnis der anderen unverändert bleibt, falls die Zusammensetzung sechs oder sieben der acht traditionellen chinesischen Arzneimittel umfasst.

16. Pharmazeutische Zusammensetzung zum Verhüten und Behandeln einer zerebrovaskulären Erkrankung in einem Patienten, umfassend mindestens die traditionellen chinesischen Arzneimittel Ren Shen, Dang Gui, Huang Qi, Gan Cao, Tian Zhu Huang und Huang Qin als Wirkstoffe.

17. Pharmazeutische Zusammensetzung nach Anspruch 16, außerdem umfassend Chai Hu.

18. Pharmazeutische Zusammensetzung nach Anspruch 17, außerdem umfassend Huang Lian.

19. Pharmazeutische Zusammensetzung nach Anspruch 16, 17 oder 18, wobei die traditionellen chinesischen Arzneimittel in Form eines trockenen Pulvers vorliegen.

20. Pharmazeutische Zusammensetzung nach Anspruch 19, wobei die traditionellen chinesischen Arzneimittel in einem Gewichtsverhältnis von Ren Shen : Dang Gui : Huang Qi : Gan Cao : Chai Hu : Huang Lian : Tian Zhu Huang : Huang Qin = 140±10 % : 166±10 % : 180±10 % : 67±10 %: 140±10 % : 120±10 % : 120±10 % : 67±10 % vorliegen, wobei die Mengen von einem oder zwei der traditionellen chinesischen Arzneimittel Null sind, während das Gewichtsverhältnis der anderen unverändert bleibt, falls die Zusammensetzung sechs oder sieben der acht traditionellen chinesischen Arzneimittel umfasst.

21. Pharmazeutische Zusammensetzung nach Anspruch 16, wobei die zerebrovaskuläre Erkrankung eine ischämische zerebrovaskuläre Erkrankung ist.

22. Zusammensetzung nach den Ansprüchen 5, 10, 15 oder 20, welche oral verabreicht werden soll.

23. Verwendung von traditionellen chinesischen Arzneimitteln bei der Herstellung eines Medikaments zur Verhütung und Behandlung einer zerebrovaskulären Erkrankung, wobei die traditionellen chinesischen Arzneimittel mindestens sechs der folgenden acht traditionellen chinesischen Arzneimittel umfassen: Ren Shen, Dang Gui, Huang Qi, Gan Cao, Chai Hu, Huang Lian, Tian Zhu Huang, Huang Qin.

24. Verwendung nach Anspruch 23, wobei die traditionellen chinesischen Arzneimittel mindestens sieben der acht traditionellen chinesischen Arzneimittel umfassen.

25. Verwendung nach Anspruch 24, wobei die traditionellen chinesischen Arzneimittel die acht traditionellen chinesischen Arzneimittel umfassen.

26. Verwendung nach Anspruch 23, 24 oder 25, wobei die acht traditionellen chinesischen Arzneimittel in Form eines trockenen Pulvers vorliegen.

27. Verwendung nach Anspruch 26, wobei die traditionellen chinesischen Arzneimittel in einem Gewichtsverhältnis von Ren Shen : Dang Gui : Huang Qi : Gan Cao : Chai Hu : Huang Lian : Tian Zhu Huang : Huang Qin = 140±10 % : 166±10 % : 180±10 % : 67±10 %: 140±10 % : 120±10 % : 120±10 % : 67±10 % vorliegen, wobei die Mengen von einem oder zwei der traditionellen chinesischen Arzneimittel Null sind, während das Gewichtsverhältnis der anderen unverändert bleibt, falls die Zusammensetzung sechs oder sieben der acht traditionellen chinesischen Arzneimittel umfasst.

28. Verwendung nach Anspruch 23, wobei die zerebrovaskuläre Erkrankung eine ischämische zerebrovaskuläre Erkrankung ist.

29. Verwendung nach Anspruch 28, wobei das Medikament oral verabreicht werden soll.

## Revendications

1. Composition de remèdes chinois traditionnels comprenant au moins Ren Shen, Dang Gui, Huang Qi, Gan Cao, Tian Zhu Huang et Huang Qin.

2. Composition selon la revendication 1, comprenant en outre Chai Hu.

3. Composition selon la revendication 2, comprenant en outre Huang Lian.

4. Composition selon la revendication 1, 2 ou 3, dans laquelle lesdits remèdes chinois traditionnels sont sous la forme d'une poudre sèche.

5. Composition selon la revendication 4, dans laquelle lesdits remèdes chinois traditionnels sont dans un rapport en poids de Ren Shen: Dang Gui: Huang Qi: Gan Cao: Chai Hu: Huang Lian : Tian Zhu Huang : Huang Qin = 140 ± 10 % : 166 ± 10 % : 180 ± 10 % . 67 ± 10 % : 140 ± 10 % : 120 ± 10 % : 120 ± 10 % : 67 ± 10 %, et dans laquelle les quantités d'un ou deux desdits remèdes chinois traditionnels sont de zéro, alors que le rapport en poids des autres reste inchangé, dans le cas où ladite composition comprend six ou sept desdits huit remèdes chinois traditionnels.

6. Composition pharmaceutique pour inhiber l'agrégation des plaquettes, comprenant au moins les remèdes chinois traditionnels Ren Shen, Dang Gui, Huang Qi, Gan Cao, Tian Zhu Huang et Huang Qin en tant qu'ingrédients actifs.

7. Composition pharmaceutique selon la revendication 6, comprenant en outre Chai Hu.

8. Composition pharmaceutique selon la revendication 7, comprenant en outre Huang Lian.

9. Composition pharmaceutique selon la revendication 6, 7 ou 8, dans laquelle lesdits remèdes chinois traditionnels sont sous la forme d'une poudre sèche.

10. Composition pharmaceutique selon la revendication 9, dans laquelle lesdits remèdes chinois traditionnels sont dans un rapport en poids de Ren Shen : Dang Gui : Huang Qi : Gan Cao : Chai Hu : Huang Lian : Tian Zhu Huang : Huang Qin = 140 ± 10 % : 166 ± 10 % : 180 ± 10 % : 67 ± 10 % : 140 ± 10 % : 120 ± 10 % : 120 ± 10 % : 67 ± 10 %, et dans laquelle les quantités d'un ou deux desdits remèdes chinois traditionnels sont de zéro, alors que le rapport en poids des autres reste inchangé, dans le cas où ladite composition comprend six ou sept desdits huit remèdes chinois traditionnels.

11. Composition pharmaceutique pour prolonger le temps de saignement chez un patient, comprenant au moins les remèdes chinois traditionnels Ren Shen, Dang Gui, Huang Qi, Gan Cao, Tian Zhu Huang et Huang Qin en tant qu'ingrédients actifs.

12. Composition pharmaceutique selon la revendication 11, comprenant en outre Chai Hu.

13. Composition pharmaceutique selon la revendication 12, comprenant en outre Huang Lian.

14. Composition pharmaceutique selon la revendication 11, 12 ou 13, dans laquelle lesdits remèdes chinois traditionnels sont sous la forme d'une poudre sèche.

15. Composition pharmaceutique selon la revendication 14, dans laquelle lesdits remèdes chinois traditionnels sont dans un rapport en poids de Ren Shen : Dang Gui : Huang Qi : Gan Cao : Chai Hu : Huang Lian : Tian Zhu Huang : Huang Qin = 140 ± 10 % : 166 ± 10 % : 180 ± 10 % : 67 ± 10 % : 140 ± 10 % : 120 ± 10 % : 120 ± 10 % : 67 ± 10 %, et dans laquelle les quantités d'un ou deux desdits remèdes chinois traditionnels sont de zéro, alors que le rapport en poids des autres reste inchangé, dans le cas où ladite composition comprend six ou sept desdits huit remèdes chinois traditionnels.

16. Composition pharmaceutique pour prévenir et traiter une maladie cérébrovasculaire chez un patient, comprenant au moins les remèdes chinois traditionnels Ren Shen, Dang Gui, Huang Qi, Gan Cao, Tian Zhu Huang et Huang Qin en tant qu'ingrédients actifs.

17. Composition pharmaceutique selon la revendication 16, comprenant en outre Chai Hu.

18. Composition pharmaceutique selon la revendication 17, comprenant en outre Huang Lian.

19. Composition pharmaceutique selon la revendication 16, 17 ou 18, dans laquelle lesdits remèdes chinois traditionnels sont sous la forme d'une poudre sèche.

20. Composition pharmaceutique selon la revendication 19, dans laquelle lesdits remèdes chinois traditionnels sont dans un rapport en poids de Ren Shen : Dang Gui : Huang Qi : Gan Cao : Chai Hu : Huang Lian : Tian Zhu Huang : Huang Qin = 140 ± 10 % : 166 ± 10 % : 180 ± 10 % : 67 ± 10 % : 140 ± 10 % : 120 ± 10 % : 120 ± 10 % : 67 ± 10 %, et dans laquelle les quantités d'un ou deux desdits remèdes chinois traditionnels sont de zéro, alors que le rapport en poids des autres reste inchangé, dans le cas où ladite composition comprend six ou sept desdits huit remèdes chinois traditionnels.

21. Composition pharmaceutique selon la revendication 16, dans laquelle ladite maladie cérébrovasculaire est une maladie cérébrovasculaire ischémique.

22. Composition selon la revendication 5, 10, 15 ou 20 qui est à administration par voie orale.

23. Utilisation de remèdes chinois traditionnels dans la fabrication d'un médicament pour prévenir et traiter une maladie cérébrovasculaire, lesdits remèdes chinois traditionnels comprenant au moins six des huit remèdes chinois traditionnels suivants : Ren Shen, Dang Gui, Huang Qi, Gan Cao, Chai Hu, Huang Lian, Tian Zhu Huang et Huang Qin.

24. Utilisation selon la revendication 23, dans laquelle lesdits remèdes chinois traditionnels comprennent au moins sept desdits huit remèdes chinois traditionnels.

25. Utilisation selon la revendication 24, dans laquelle lesdits remèdes chinois traditionnels comprennent lesdits huit remèdes chinois traditionnels.

26. Utilisation selon la revendication 23, 24 ou 25, dans laquelle lesdits huit remèdes chinois traditionnels sont sous la forme d'une poudre sèche.

27. Utilisation selon la revendication 26, dans laquelle lesdits remèdes chinois traditionnels sont dans un rapport en poids de Ren Shen : Dang Gui : Huang Qi : Gan Cao : Chai Hu : Huang Lian : Tian Zhu Huang : Huang Qin = 140 ± 10 % : 166 ± 10 % : 180 ± 10 % : 67 ± 10 % : 140 ± 10 % : 120 ± 10 % : 120 ± 10 % : 67 ± 10 %, et dans laquelle les quantités d'un ou deux desdits remèdes chinois traditionnels sont de zéro, alors que le rapport en poids des autres reste inchangé, dans le cas où ladite composition comprend six ou sept desdits huit remèdes chinois traditionnels.

28. Utilisation selon la revendication 23, dans laquelle ladite maladie cérébrovasculaire est une maladie cérébrovasculaire ischémique.

29. Utilisation selon la revendication 28, dans laquelle ledit médicament est à administration par voie orale.
